# EUROPEAN PATENT APPLICATION

(11) **EP 0 695 762 A1**
(43) Date of publication of application: **07.02.1996**
(21) Application number: 94907678.0
(22) Date of filing: 24.02.1994
(51) Int. Cl.: C08F 2/32

(54) **METHOD OF MANUFACTURING POLYMER PARTICLES OF INDETERMINATE FORM**

(30) Priority: 01.03.1993 JP 40182/93
(71) Applicant: Kao Corporation, Chuo-Ku Tokyo 103 (JP)
(72) Inventor: MIYANAGA, Seiichi, Wakayama-shi, Wakayama 640 (JP); INA, Yoshimitsu, Wakayama-shi, Wakayama 641 (JP); MINAMI, Takahide, Wakayama-shi, Wakayama 640 (JP); AMIYA, Takayuki, Wakayama-shi, Wakayama 649-63 (JP)
(74) Representative: Grünecker, Kinkeldey, Stockmair & Schwanhäusser Anwaltssozietät
(86) International application number: JP9400295
(87) International publication number: WO9420543

(57) **Abstract**

This invention provides a method of manufacturing polymer particles of indeterminate form, characterized in that a cationic surface active agent or an amphoteric surface active agent is present as a dispersant when a water-soluble polymerizable monomer is polymerized by using an inert hydrophobic organic solvent and an aqueous solution of the water-soluble polymerizable monomer. According to the manufacturing method of the present invention, polymer particles of indeterminate form useful as a water absorbing resin having a low apparent specific gravity, a high water absorption degree including a high initial water absorption rate, a high gas-permeability, a high liquid-permeability and a high post-water-absorption gel strength, and, especially, as a water absorbing resin for the production of an absorption body for a sanitary material contacting a human body, such as a sanitary napkin, a paper diaper, shorts for adults, a tampon and sanitary cotton can be obtained by a simple operation with a high productivity and on a mass-production basis.

## Description

### Technical Field

This invention relates to a process for producing polymer particles of irregular shape which have a small bulk density and excellent water absorbability, air permeability and liquid permeability, and exhibits high gel strength after water absorption and are therefore useful as water-absorbing resin.

### Background Art

Water-absorbing resins have widely been used for their water absorbing and retaining properties in the medical field (e.g., as sanitary materials), the food industrial field, and the agricultural and horticultural field. When applied particularly to sanitary products such as sanitary napkins and disposable diapers, water-absorbing resins are required to have not only a high water absorption per unit weight but a high rate of water absorption. The water absorption per unit weight depends on the molecular structure of the resin. With the weight being equal, as the particle size of a powdered resin is reduced, the surface area increases, and the rate of water absorption is considered to increase at the same time. Accordingly, there have been various proposals on production of water-absorbing resins having a molecular structure adequate to water absorption and a small particle size.

For example, Japanese Patent Application Laid-Open 57-167302 discloses a process in which a specific surface active agent is used in the polymerization as a dispersion stabilizer thereby to provide a water-absorbing resin as fine particles (1 to 40 µm) having an increased rate of absorption. However, water-absorbing resin particles with their size merely reduced form lumps of powder in the course of water absorption, failing to achieve a sufficient speed of water absorption.

Japanese Patent Application Laid-Open 62-106902 discloses a process for producing a water-absorbing porous polymer having voids in the inside thereof and a large surface area, which comprises preparing an O/W/O emulsion of a monomer and polymerizing the monomer. However, the process is disadvantageous in that the step for preparing an O/W/O emulsion is complicated and that the voids of the resulting polymer are not always continuous for obtaining a sufficiently high initial rate of absorption.

On the other hand, Japanese Patent Application Laid-Open 61-200102 proposes a process comprising initiating water-in-oil reversed phase suspension polymerization at 0 to 20°C and, after maintaining the polymerization temperature until a polymerization rate of 30% is reached, raising the temperature to complete the polymerization, thereby obtaining water-absorbing resin particles having a high void content and a high rate of water absorption in which fine particles of 1 to 40 µm are bonded together relatively loosely. In this process, it is necessary to control the polymerization temperature within the range of from 0 to 20°C until the rate of polymerization reaches 30%. It is extremely difficult, however, to efficiently remove the heat of polymerization to keep the polymerization temperature at such a low level, so that the process is not suited to mass production. Additionally, the process is not efficient because of involvement of considerable adhesion of the polymer produced to the polymerization vessel during the polymerization.

Accordingly, an object of the present invention is to provide a process for producing irregular-shaped polymer particles which have a small bulk density and excellent water absorbability, air permeability and liquid permeability, and exhibits high gel strength after water absorption and are therefore useful as water-absorbing resin; which process can be carried out through simple operation, achieves excellent productivity, and is applicable to mass production.

### Disclosure of the Invention

As a result of extensive investigations, the present inventors have found that the above object of the present invention is accomplished by using a specific surface active agent as a dispersant in polymerization of a water-soluble polymerizable monomer.

The present invention has been completed based on the above finding. It provides a process for producing irregular-shaped polymer particles comprising polymerizing a water-soluble polymerizable monomer by using an aqueous solution of the water-soluble polymerizable monomer and a hydrophobic organic solvent inert to the polymerization, characterized in that the polymerization is carried out in the presence of a cationic or amphoteric surface active agent as a dispersant.

It is preferable that the dispersant is a surface active agent having an ammonium group.

It is preferable that the dispersant has a molecular weight of not more than 1000.

It is preferable that the surface active agent is a compound represented by formula (I): 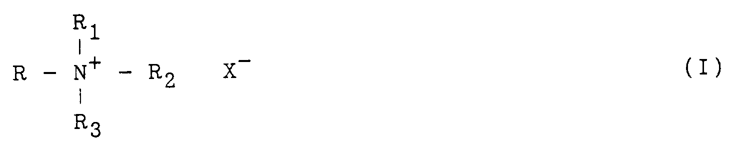
wherein R represents an alkyl, alkenyl or alkylaryl group having 6 or more carbon atoms; R₁, R₂, and R₃ independently represent a hydrogen atom or an alkyl, alkenyl, alkylaryl or hydroxyalkyl group having 1 to 8 carbon atoms, or two or all the groups of R₁, R₂, and R₃ may be connected together to form a saturated or unsaturated ring; and X⁻ represents a halide ion, such as a chloride ion, a bromide ion or an iodide ion, a hydroxide ion (OH⁻) or an acid ion, such as a sulfate ion, an alkylsulfate ion, an alkylsulfonate ion or an organic carboxylate ion.

It is preferable that the surface active agent is a compound represented by formula (II): 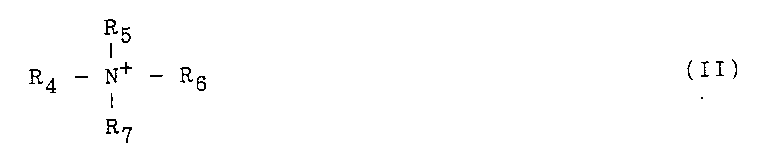
wherein R₄ represents an alkyl, alkenyl or alkylaryl group having 6 or more carbon atoms; R₅ and R₆ independently represent a hydrogen atom or an alkyl, alkenyl, alkylaryl or hydroxyalkyl group having 1 to 8 carbon atoms, or R₅ and R₆ may be connected together to form a saturated or unsaturated ring; and R₇ represents a carboxyalkyl, sulfoalkyl or phosphoalkyl group having 1 to 8 carbon atoms or an oxygen atom.

It is preferable that the surface active agent is a compound represented by formula (III): 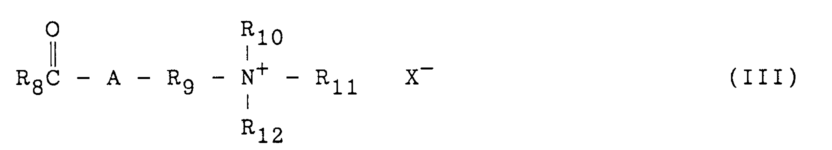
wherein R₈C=O represents an acyl group having 6 or more carbon atoms; A represents -O- or 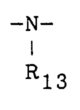
(wherein R₁₃ represents an alkyl group having 1 to 3 carbon atoms); R₉ represents an alkylene group having 1 to 4 carbon atoms; R₁₀, R₁₁, and R₁₂ independently represent a hydrogen atom or an alkyl, alkenyl or alkylaryl group having 1 to 8 carbon atoms, or two or all the groups of R₁₀, R₁₁, and R₁₂ may be connected together to form a saturated or unsaturated ring; and X⁻ represents a halide ion, such as a chloride ion, a bromide ion or an iodide ion, a hydroxide ion (OH⁻) or an acid ion, such as a sulfate ion, an alkylsulfate ion, an alkylsulfonate ion or an organic carboxylate ion.

It is preferable that the surface active agent is a compound represented by formula (IV): 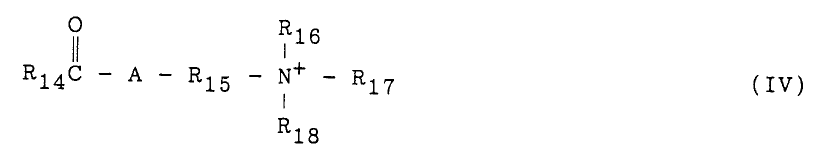
wherein R₁₄C=O represents an acyl group having 6 or more carbon atoms; A represents -O- or 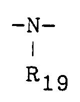
(wherein R₁₉ represents an alkyl group having 1 to 3 carbon atoms); R₁₅ represents an alkylene group having 1 to 4 carbon atoms; R₁₆ and R₁₇ independently represent a hydrogen atom or an alkyl, alkenyl or alkylaryl group having 1 to 8 carbon atoms, or R₁₆ and R₁₇ may be connected together to form a saturated or unsaturated ring; and R₁₈ represents a carboxyalkyl, sulfoalkyl or phosphoalkyl group having 1 to 8 carbon atoms or an oxygen atom.

It is preferable that the dispersant has a sulfonium group or a phosphonium group and has a molecular weight of not more than 1000.

It is preferable that the water-soluble polymerizable monomer is an olefinic unsaturated carboxylic acid or an alkali salt thereof.

The irregular-shaped polymer particles obtained by the process of the present invention are irregular-shaped polymer particles which have a non-spherical shape, an average particle size of not less than 10 µm as measured by sieving (specified by JIS), and a high void content. Therefore, the irregular-shaped polymer particles have a small bulk density, are excellent in water absorbability (e.g., initial rate of water absorption), air permeability, liquid permeability, and gel strength after water absorption and are useful as water-absorbing resin.

According to the present invention, the above-mentioned irregular-shaped polymer particles can be produced through simple operation with excellent productivity by polymerizing a water-soluble polymerizable monomer in the presence of a cationic or amphoteric surface active agent, and the process of the present invention is capable of mass production.

### Brief Explanation of Drawings

Fig. 1 is a schematic illustration of an apparatus used in Examples and Comparative Examples for measuring a water absorption representing a rate of water absorption.

Fig. 2 is a schematic illustration of an apparatus used in Examples and Comparative Examples for measuring a rate of permeation to physiological saline.

### Best Mode for Carrying out the Invention

The process of the present invention for producing irregular-shaped polymer particles is a process in which polymerization of a water-soluble polymerizable monomer is conducted in the presence of a cationic or amphoteric surface active agent as a dispersant.

The present invention will be explained below in detail. Unless otherwise specified, all the percents are by weight.

The water-soluble polymerizable monomer which can be used in the present invention preferably includes vinyl monomers having a polymerizable unsaturated group, such as olefinic unsaturated carboxylic acids or salts thereof, olefinic unsaturated carboxylic acid esters, olefinic unsaturated sulfonic acids or salts thereof, olefinic unsaturated phosphoric acids or salts thereof, olefinic unsaturated amines, olefinic unsaturated ammonium salts, and olefinic unsaturated amides. Preferred of them are olefinic unsaturated carboxylic acids or salts thereof.

The olefinic unsaturated carboxylic acids or salts thereof include acrylic acid, methacrylic acid, maleic acid, fumaric acid, or alkali salts of these acids. The olefinic unsaturated carboxylic esters include methoxypolyethylene glycol (meth)acrylate, phenoxypolyethylene glycol (meth)acrylate, and hydroxyethyl (meth)acrylate. The olefinic unsaturated sulfonic acids or salts thereof include (meth) acrylamidomethylpropanesulfonic acid, allylsulfonic acid or alkali salts thereof. The olefinic unsaturated phosphoric acids or salts thereof include (meth)acryloyl(poly)oxyethylenephosphoric esters or alkali salts thereof. The olefinic unsaturated amines include dimethylaminoethyl (meth)acrylate. The olefinic unsaturated ammonium salts include (meth)acryloyloxyethylenetrimethylammonium halides. The olefinic unsaturated amides include (meth)acrylamide; (meth)acrylamide derivatives, such as N-methyl(meth)acrylamide, N-ethyl(meth)acrylamide, and N-propyl(meth)acrylamide; and vinylmethylacetamide. These monomers may be used either individually or as a mixture of two or more thereof. The alkali salts as referred to above include alkali metal salts, alkaline earth metal salts, and ammonium salts.

The aqueous solution of the water-soluble polymerizable monomer preferably has a monomer concentration of from 1 to 90%, still preferably of from 10 to 60%.

The inert hydrophobic organic solvent which can be used in the polymerization includes aliphatic hydrocarbons, such as n-pentane, cyclopentane, n-hexane, cyclohexane, n-heptane, and methylcyclohexane; aromatic hydrocarbons, such as benzene and toluene; aliphatic alcohols having 4 to 6 carbon atoms, such as n-butyl alcohol and n-amyl alcohol; aliphatic ketones, such as methyl ethyl ketone; and aliphatic esters, such as ethyl acetate. These solvents may be used either individually or as a combination of two or more thereof.

The hydrophobic organic solvent is preferably used in an amount of 50 to 500% based on the aqueous solution of the water-soluble polymerizable monomer.

The cationic or amphoteric surface active agent (hereinafter simply referred to as "surface active agent") which can be used in the present invention as a dispersant includes those having an ammonium group, a sulfonium group or a phosphonium group.

The surface active agents having an ammonium group preferably include those represented by the following formulae (I), (II), (III) or (IV). 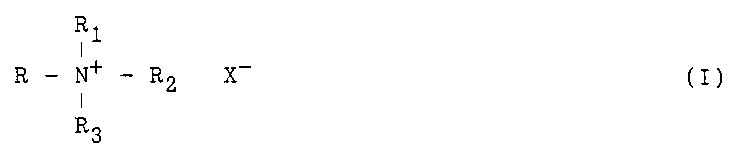
wherein R represents an alkyl, alkenyl or alkylaryl group having 6 or more carbon atoms; R₁, R₂, and R₃ independently represent a hydrogen atom or an alkyl, alkenyl, alkylaryl or hydroxyalkyl group having 1 to 8 carbon atoms, or two or all the groups of R₁, R₂, and R₃ may be connected together to form a saturated or unsaturated ring; and X⁻ represents a halide ion, such as a chloride ion, a bromide ion or an iodide ion, a hydroxide ion (OH⁻) or an acid ion, such as a sulfate ion, an alkylsulfate ion, an alkylsulfonate ion or an organic carboxylate ion. 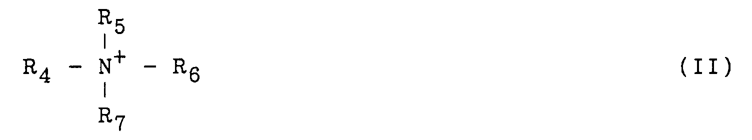
wherein R₄ represents an alkyl, alkenyl or alkylaryl group having 6 or more carbon atoms; R₅ and R₆ independently represent a hydrogen atom or an alkyl, alkenyl, alkylaryl or hydroxyalkyl group having 1 to 8 carbon atoms, or R₅ and R₆ may be connected together to form a saturated or unsaturated ring; and R₇ represents a carboxyalkyl, sulfoalkyl or phosphoalkyl group having 1 to 8 carbon atoms or an oxygen atom. 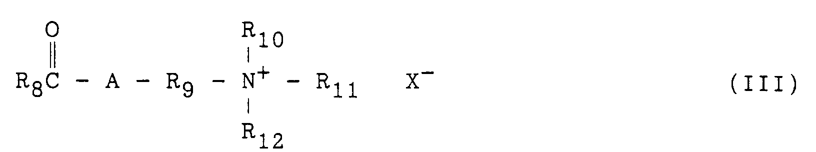
wherein R₈C=O represents an acyl group having 6 or more carbon atoms; A represents -O- or 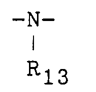
(wherein R₁₃ represents an alkyl group having 1 to 3 carbon atoms); R₉ represents an alkylene group having 1 to 4 carbon atoms; R₁₀, R₁₁, and R₁₂ independently represent a hydrogen atom or an alkyl, alkenyl or alkylaryl group having 1 to 8 carbon atoms, or two or all the groups of R₁₀, R₁₁, and R₁₂ may be connected together to form a saturated or unsaturated ring; and X⁻ represents a halide ion, such as a chloride ion, a bromide ion or an iodide ion, a hydroxide ion (OH⁻) or an acid ion, such as a sulfate ion, an alkylsulfate ion, an alkylsulfonate ion or an organic carboxylate ion. 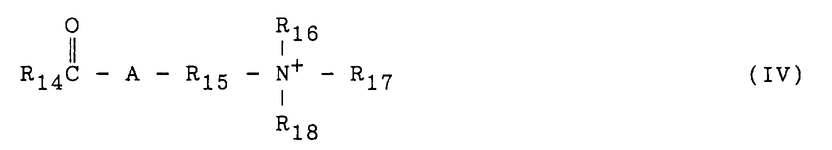
wherein R₁₄C=O represents an acyl group having 6 or more carbon atoms; A represents -O- or 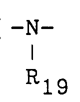
(wherein R₁₉ represents an alkyl group having 1 to 3 carbon atoms); R₁₅ represents an alkylene group having 1 to 4 carbon atoms; R₁₆ and R₁₇ independently represent a hydrogen atom or an alkyl, alkenyl or alkylaryl group having 1 to 8 carbon atoms, or R₁₆ and R₁₇ may be connected together to form a saturated or unsaturated ring; and R₁₈ represents a carboxyalkyl, sulfoalkyl or phosphoalkyl group having 1 to 8 carbon atoms or an oxygen atom.

These surface active agents preferably have a molecular weight of not more than 1000.

In formula (I), the ammonium group is preferably a quaternary ammonium group, and R is preferably an alkyl, alkenyl or alkylaryl group having 6 to 22 carbon atoms. In formula (II), the ammonium group is preferably a quaternary ammonium group, and R₄ is preferably an alkyl, alkenyl or alkylaryl group having 6 to 22 carbon atoms.

In formula (III), R₈C=O is preferably an acyl group having 6 to 22 carbon atoms. In formula (IV), R₁₄C=O is preferably an acyl group having 6 to 22 carbon atoms.

Examples of the surface active agents represented by formula (I) are trimethylstearylammonium chloride, trimethylcetylammonium chloride, dimethylbenzylstearylammonium chloride , dimethylbenzylcetylammonium chloride, dimethylallylcetylammonium chloride, dimethylallylstearylammonium chloride, stearylpyridinium chloride, and cetylpyridinium chloride.

Examples of the surface active agents represented by formula (II) are carboxymethyldimethylcetylammonium , carboxymethyldimethylstearylammonium, sulfopropyldimethylcetylammonium, sulfopropyldimethylstearylammonium, phosphomethyldimethylcetylammonium, phosphomethyldimethylstearylammonium, and lauryldimethylamine oxide.

Examples of the surface active agents represented by formula (III) are trimethyl(dodecanoamidopropyl)ammonium chloride, trimethyl(hexadecanoamidopropyl)ammonium chloride, and trimethyl(octadecanoamidopropyl)ammonium chloride.

Examples of the surface active agents represented by formula (IV) are carboxymethyldimethyl(dodecanoamidopropyl)ammonium, carboxymethyldimethyl(tetradecanoamidopropyl)ammonium, carboxymethyldimethyl(hexadecanoamidopropyl)ammonium, carboxymethyldimethyl(octadecanoamidopropyl)ammonium, and dimethylsulfomethyl(octadecanoamidopropyl)ammonium.

The surface active agents having a sulfonium group include those represented by the following formula (V), (VI), (VII), (VIII), (IX) or (X); and the surface active agents having a phosphonium group include those represented by the following formula (XI). These surface active agents preferably have a molecular weight of not more than 1000.

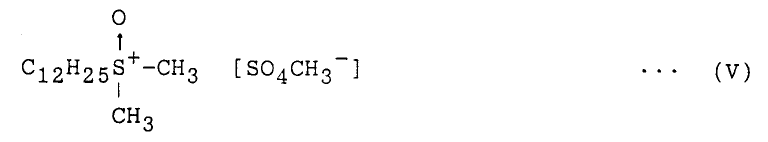

C₁₆H₃₃(C₂H₅)(CH₃)S⁺X⁻ (VI)

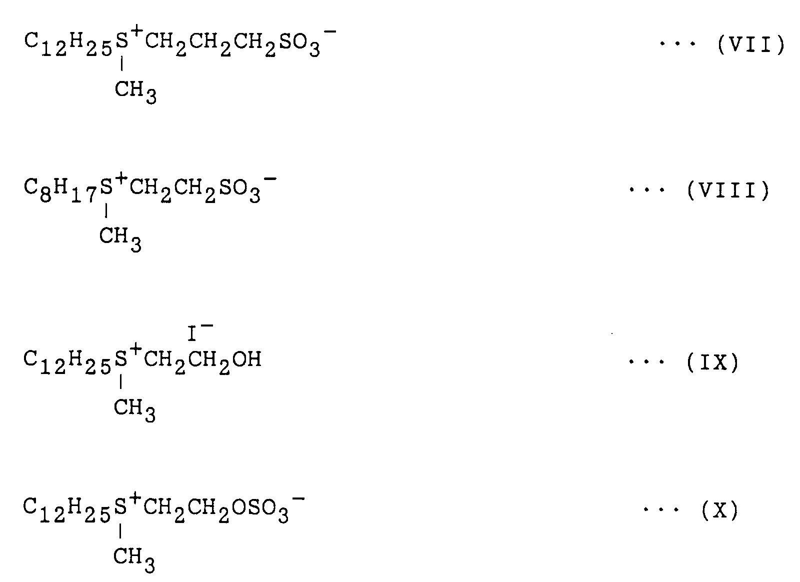

(C₆H₅)₃P⁺CH₂CH₂CH₂SO₃⁻ (XI)

In formula (VI), X⁻ represents a halide ion, e.g., a chloride ion, a bromide ion or an iodide ion.

The above-described surface active agents include amine compounds capable of forming an ammonium salt in the polymerization system to serve as a cationic or amphoteric surface active agent.

While these surface active agents produce a sufficient effect when used individually, they may be used as a combination of two or more thereof.

The surface active agent exerts its effect even at a small amount. A suitable amount of the surface active agent to be used is from 0.01 to 10%, preferably from 0.02 to 5%, based on the water-soluble polymerizable monomer. If the amount is less than 0.01%, the effect is hardly produced. Addition of more than 10% is of no economical advantage.

Polymerization of the water-soluble polymerizable monomer using the aforesaid hydrophobic organic solvent and an aqueous solution of the water-soluble polymerizable monomer can be carried out according to, for example, methods (1) to (4) described below.
(1) A method in which an aqueous solution of a water-soluble polymerizable monomer and a hydrophobic organic solvent are mixed all at once, followed by polymerization (all-at-once polymerization).
(2) A method in which an aqueous solution of a water-soluble polymerizable monomer is added dropwise to a hydrophobic organic solvent to effect polymerization successively (successive polymerization).
(3) A method in which an aqueous solution of a water-soluble polymerizable monomer is previously mixed or dispersed in a part of a hydrophobic organic solvent, and the resulting mixture is added dropwise to the rest of the hydrophobic organic solvent to conduct polymerization (pre-dispersion).
(4) A combination of methods (1) to (3).

The surface active agent is added to the polymerization system as a dispersant in accordance with, for example, the following manners (1) to (4):
(1) The surface active agent is previously dispersed in the hydrophobic organic solvent.
(2) The surface active agent is previously dissolved or dispersed in an aqueous solution of the water-soluble polymerizable monomer.
(3) The surface active agent is slowly added to the system under polymerization.
(4) A combination of (1) to (3).

The polymerization is preferably performed in the presence of a polymerization initiator. The polymerization initiator to be used is not particularly limited as long as it is a water-soluble radical initiator. Preferable polymerization initiators include ketone peroxides, e.g., methyl ethyl ketone peroxide and methyl isobutyl ketone peroxide; dialkyl peroxides, e.g., di-t-butyl peroxide and t-butylcumyl peroxide; alkyl peroxy esters, e. g. , t-butyl peroxyacetate, t-butyl peroxyisobutyrate, and t-butyl peroxypivalate; hydrogen peroxide; persulfates, e.g., potassium persulfate and ammonium persulfate; perchlorates, e.g., potassium perchlorate and sodium perchlorate; halogenic acid salts, e.g., potassium chlorate and potassium bromate; and azo compounds, e.g., 2-(carbamoylazo)isobutyronitrile, 2,2-azobis(N,N'-dimethyleneisobutylamidine) dihydrochloride, 2,2'-azobis(2-amidinopropane) dihydrochloride, 2,2'-azobis(N,N'-dimethyleneisobutylamidine), 4,4'-azobis(4-cyanopentanoic acid), azobisisobutyronitrile, 2,2'-azobis (4-methoxy-2,4-dimethylvaleronitrile), (1-phenylethyl)azodiphenylmethane, dimethyl 2,2'-azobisisobutyrate, 2,2'-azobis(2-methylbutyronitrile), 1,1'-azobis(1-cyclohexanecarbonitrile), 2,2'-azobis(2,4,4'-trimethylpentane), 2-phenylazo-2,4-dimethyl-4-methoxyvaleronitrile, and 2,2'-azobis(2-methylpropane). These initiators may be used either individually or as a combination of two or more thereof.

The polymerization initiator is used in an amount usually of from 0.01 to 10%, and preferably of from 0.02 to 5%, based on the water-soluble polymerizable monomer.

While not limiting, the initiator is preferably added to an aqueous solution of the water-soluble polymerizable monomer beforehand.

The polymerization temperature is not particularly limited, as long as falling within a temperature range allowing the water-soluble polymerizable monomer used to be polymerised. From the standpoint of speed and rate of polymerization, the polymerization temperature is preferably selected from the range optimum for the particular water-soluble polymerizable monomer used. For example, a range of from 20 to 150°C, particularly 40 to 100°C, is preferred for (meth)acrylic acid or a salt thereof. If the temperature exceeds 150°C, the degree of crosslinking extremely increases, resulting in an extreme reduction of water absorbability of the resulting polymer particles. If it is lower than 20°C, the speed of polymerization is extremely reduced.

In the present invention, while it is desirable that one of the above-mentioned water-soluble polymerizable monomers is homopolymerized or two or more of them are copolymerized, water-insoluble monomers copolymerizable with the water-soluble polymerizable monomer(s) may be used in combination in a proportion of not higher than 50% based on the total monomers. Such copolymerizable water-insoluble monomers include esters between an unsaturated carboxylic acid, e.g., acrylic acid, methacrylic acid, maleic acid or fumaric acid, and an alkyl group having 1 to 2 carbon atoms.

In addition to the above-mentioned hydrophobic solvent, amphiphilic solvents may be used in an amount not exceeding the amount of the hydrophobic solvent. Examples of usable amphiphilic solvents include alcohols, such as methanol, ethanol, propanol, and 2-propanol; ketones, such as acetone; and ethers, such as diethyl ether, dipropyl ether, tetrahydrofuran, and dioxane.

In addition to the above-mentioned surface active agents, nonionic surface active agents, anionic surface active agents, other amphoteric surface active agents, other cationic surface active agents, high polymeric dispersants, and the like may be used in an amount of not more than 100 parts by weight per 100 parts by weight of the above-mentioned surface active agents of the present invention.

In polymerizing the water-soluble polymerizable monomer for obtaining irregular-shaped polymer particles having a small bulk density, excellent in water absorbability (e.g., initial rate of water absorption), air permeability, liquid permeability, and gel strength after water absorption and useful as water-absorbing resin, it is preferable to add a known crosslinking agent to the polymerization system. The crosslinking agent can be added at any stage before, during or after the polymerization.

Examples of the crosslinking agents include polyallyl compounds, such as N,N-diallyl(meth)acrylamide, diallylamine, diallylmethacrylamine, diallyl phthalate, diallyl maleate, diallyl terephthalate, triallyl cyanurate, and triallyl phosphate; polyvinyl compounds, such as divinylbenzene, N,N-methylenebisacrylamide, ethylene glycol diacrylate, ethylene glycol dimethacrylate, and glycerin trimethacrylate; polyglycidyl ethers, such as ethylene glycol diglycidyl ether, polyethylene glycol diglycidyl ether, and polyglycerin polyglycidyl ether; halo-epoxy compounds, such as epichlorohydrin and α-methylchlorohydrin; polyaldehydes, such as glutaraldehyde and glyoxal; polyols, such as glycerin; polyamines, such as ethylenediamine; hydroxyvinyl compounds, such as 2-hydroxyethyl methacrylate; and inorganic or organic metal salts capable of providing a polyvalent ion, such as a calcium ion, a magnesium ion, a zinc ion or an aluminum ion.

Modifiers, such as phenol polyoxyethylene glycol ether, can also be used.

The amount of the crosslinking agent or modifier is arbitrarily selected according to the desired properties of the final product polymer. They are usually used in an amount of 0.01 to 10% based on the polymer produced.

After the polymerization, the resulting polymer is dried by means of a vacuum drier, a fluidized bed drier, etc. either directly or after removal of the solvent by decantation or centrifugation, and if desired ground or granulated to obtain irregular-shaped polymer particles.

### EXAMPLES

The present invention is hereinafter described in greater detail with reference to Examples and Comparative Examples, but it should be understood that the present invention is not construed as being limited thereto.

Tests in Examples and Comparative Examples were carried out according to the following test methods.

### [Measurement of Equilibrated Water Absorption on Swelling]

One gram of a polymer is dispersed in a large excess of physiological saline (0.9% aqueous solution of sodium chloride) and allowed to be swollen until the water absorption comes to equilibrium. The polymer is collected by filtration through a 80 mesh metal net and weighed. The weight of the swollen polymer (W) is divided by the weight of the polymer before water absorption (W₀) to obtain an equilibrated water absorption on swelling (W/W₀; g/g).

### [Measurement of Water Absorption Representing Rate of Water Absorption]

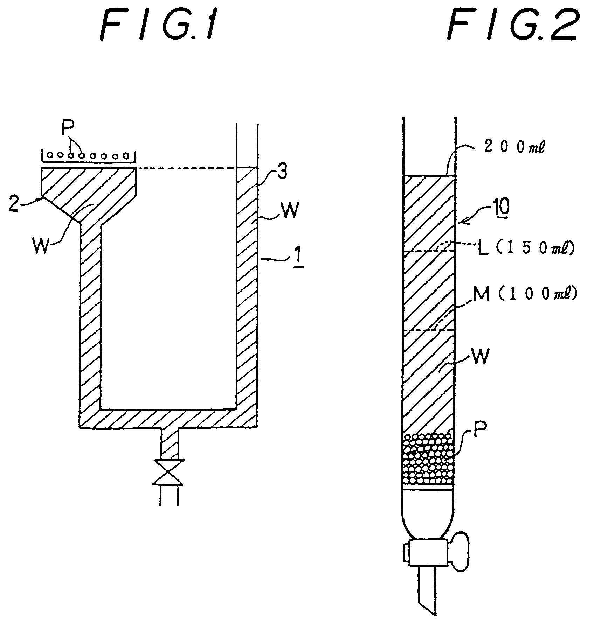

A demand wettability tester shown in Fig. 1 which is generally known as an apparatus for carrying out a DW method is used. As shown in Fig. 1, physiological saline W was put in the tester with the two liquid levels being equal, and 0.3 g of polymer P is scattered on mounting 2 (diameter: 70 mm⌀; No. 1 glass filter carrying thereon No. 2 filter paper). Taking the water absorption at the time of scattering the polymer as zero, the water absorption after 30 seconds was measured by reading the scale on the burette indicating a drop of the liquid level of physiological saline W. The measured value is taken as a water absorption (ml) representing the rate of water absorption.

### [Measurement of Rate of Physiological Saline Permeation]

In apparatus 10 shown in Fig. 2 (cylindrical glass filter (inner diameter: 25.6 mm; length of the cylindrical portion: about 500 mm) with a cock) is put 0.5 g of a polymer and swollen to equilibrium with excess physiological saline. The cock is closed with the liquid level adjusted to a height of 200 ml from the bottom. On confirming that the swollen polymer P has been sedimented sufficiently as illustrated, the cock is opened, and the time required for physiological saline W to pass through the section between two gage marks, L (150 ml high from the bottom) and M (100 ml high from the bottom), which corresponds to a 50 ml portion, is measured. The amount (ml) of the liquid between the gage marks is divided by the measured time (min) to obtain a rate of liquid permeability (ml/min).

### [Calculation of Average Particle Size]

Polymer particles weighing 100 g are classified using JIS sieves. An average particle size is calculated from the weight percent of each fraction.

### EXAMPLE 1

Acrylic acid (72.1 g) was diluted with 18.0 g of water and neutralized with 98.9 g of a 30 wt% aqueous solution of sodium hydroxide while cooling. To the solution was added 10.7 g of 2.8 wt% potassium persulfate to prepare a uniform aqueous solution of monomer/initiator.

Separately, 283 ml of cyclohexane was put to a 500 ml flask equipped with a reflux condenser, a dropping funnel, a stirring rod, and a tube for introducing nitrogen, and 0. 55 g of trimethylstearylammonium chloride was added thereto, followed by stirring at 300 rpm and dispersing. After displacing the inner atmosphere with nitrogen, the mixture was heated to 75°C, and the above-prepared monomer/initiator aqueous solution was added thereto dropwise over a period of 30 minutes. After completion of the addition, the mixture was stirred at 75°C for 1.5 hours and then at 80°C for 4 hours to conduct polymerization.

After completion of the polymerization, the product was collected and dried under reduced pressure to obtain 88.4 g of a sodium acrylate polymer as granular particles having a distorted shape, a particle size varying from 100 to 1500 µm (average particle size: 800 µm), and a bulk density of 0.54 g/ml. The surface of the individual polymer particle showed extremely remarkable unevenness, looking like irregular-shaped particles of several to 20 µm in diameter fused together.

### EXAMPLE 2

In 150 g of water was dissolved 71.1 g of acrylamide, and 10.7 g of a 2.8 wt% aqueous solution of 2,2'-azobis(2-amidinopropane) dihydrochloride was added thereto to prepare a uniform monomer/initiator aqueous solution. In the same manner as in Example 1 except for using cyclohexane as a solvent in place of hexane, 68 g of an acrylamide polymer was obtained. The resulting polymer was granular particles having a distorted shape, a particle size varying from 100 to 1400 µm (average particle size: 700 µm), and a bulk density of 0.50 g/ml. The surface of the individual polymer particle showed extremely remarkable unevenness, looking like irregular-shaped particles of several to 20 µm in diameter fused together.

### EXAMPLE 3

In 150 g of water was dissolved 106.6 g of sodium acrylamidomethylpropanesulfonate, and 10.7 g of a 2.8 wt% aqueous solution of 2,2'-azobis(2-amidinopropane) dihydrochloride was added thereto to prepare a uniform monomer/initiator aqueous solution. In the same manner as in Example 1 except for using the thus prepared monomer/initiator solution, 95 g of a sodium acrylamidomethylpropanesulfonate polymer was obtained. The resulting polymer was granular particles having a distorted shape, a particle size varying from 100 to 950 µm (average particle size: 500 µm), and a bulk density of 0.48 g/ml. The surface of the individual polymer particle showed extremely remarkable unevenness, looking like irregular-shaped particles of several to 20 µm in diameter fused together.

### EXAMPLE 4

Acrylic acid (72.1 g) was diluted with 32.0 g of water and neutralized with 98.9 g of a 30 wt% aqueous solution of sodium hydroxide while cooling. To the solution was added 10.7 g of 2.8 wt% potassium persulfate to prepare a uniform aqueous solution of monomer/initiator.

Separately, 283 ml of cyclohexane was put to a 500 ml flask equipped with a reflux condenser, a dropping funnel, a stirring rod, and a tube for introducing nitrogen and 0.55 g of trimethylstearylammonium chloride was added thereto, followed by stirring at 300 rpm and dispersing. After displacing the inner atmosphere with nitrogen, the mixture was heated to 75°C, and the above-prepared sodium acrylate monomer aqueous solution was added thereto dropwise over a period of 30 minutes. At the same time, 0.058 g of ethylene glycol diglycidyl ether as an epoxy-containing bifunctional crosslinking agent was slowly added to the mixture using a syringe. After the addition, the mixture was stirred at 75°C for 1.5 hours and then at 80°C for 4 hours to conduct polymerization.

After completion of the polymerization, the product was collected and dried under reduced pressure to obtain 88.4 g of a sodium acrylate polymer as granular particles. The polymer particles had a distorted shape, a particle size varying from 110 to 1400 µm (average particle size: 750 µm), and a bulk density of 0.53 g/ml. The surface of the individual polymer particle showed extremely remarkable unevenness, looking like irregular-shaped particles of several to 20 µm in diameter fused together. The resulting polymer was subjected to testing for measurement of an equilibrated water absorption on swelling, a water absorption representing a rate of water absorption, and a rate of permeation to physiological saline. The results obtained are shown in Table 1 below.

### EXAMPLE 5

Acrylic acid (36.1 g) was diluted with 16.0 g of water and neutralized with 53.0 g of a 30 wt% aqueous solution of sodium hydroxide while cooling. To the solution were added 101.3 g of a 40 wt% aqueous solution of sodium acrylamidomethylpropanesulfonate and then 10.7 g of a 2.8 wt% aqueous solution of potassium persulfate to prepare a uniform monomer/initiator aqueous solution. In the same manner as in Example 4 except for using the thus prepared monomer/initiator solution, 93.5 g of a sodium acrylate-sodium acrylamidomethylpropanesulfonate copolymer was obtained. The resulting polymer was granular particles having a distorted shape, a particle size varying from 80 to 1200 µm (average particle size: 550 µm), and a bulk density of 0.45 g/ml. The resulting polymer was tested in the same manner as in Example 4. The results obtained are shown in Table 1.

### EXAMPLE 6

In the same manner as in Example 4, except for replacing trimethylstearylammonium chloride used as a dispersant with carboxymethyldimethylcetylammonium, granular particles having a markedly uneven surface and a distorted shape were obtained. The polymer had a bulk density of 0.38 g/ml. The resulting polymer was tested in the same manner as in Example 4. The results obtained are shown in Table 1.

### EXAMPLE 7

Acrylic acid (72.1 g) was diluted with 18.0 g of water and neutralized with 98.9 g of a 30 wt% aqueous solution of sodium hydroxide while cooling. To the solution was added 10.7 g of a 2.8 wt% aqueous solution of potassium persulfate to prepare a uniform aqueous solution of a sodium acrylate monomer.

Separately, 283 ml of cyclohexane was put to a 500 ml flask equipped with a reflux condenser, a dropping funnel, a stirring rod, and a tube for introducing nitrogen, and 0.55 g of trimethylstearylammonium chloride was added thereto, followed by stirring at 300 rpm and dispersing. After displacing the inner atmosphere with nitrogen, the mixture was heated to 75°C, and the above-prepared sodium acrylate monomer aqueous solution was added thereto dropwise over a period of 30 minutes. After the addition, the mixture was stirred at 75°C for 1.5 hours and then at 80°C for 4 hours to conduct polymerization while driving only water out of the system from the refluxing azeotropic mixture of cyclohexane and water. When the water content of the sodium acrylate polymer gel reached 30 w%, 0.18 g of ethylene glycol diglycidyl ether as an epoxy-containing bifunctional crosslinking agent was added to the reaction mixture, and the reaction was further continued for 30 minutes. The product was collected and dried under reduced pressure to obtain 88.0 g of a partially crosslinked sodium acrylate polymer as granular particles. The polymer particles had a distorted shape, a particle size varying from 100 to 1400 µm (average particle size: 700 µm), and a bulk density of 0.42 g/ml. The resulting polymer was tested in the same manner as in Example 4. The results obtained are shown in Table 1 below.

### EXAMPLE 8

Granular particles having a remarkably uneven surface and a distorted shape were obtained in the same manner as in Example 7, except for using carboxymethyldimethyl(dodecanoamidopropyl)ammonium in place of trimethylstearylammonium chloride. The bulk density was 0.41 g/ml. The resulting polymer was tested in the same manner as in Example 4. The results obtained are shown in Table 1.

### EXAMPLE 9

Acrylic acid (72.1 g) was diluted with 18.0 g of water and neutralized with 98.9 g of a 30 wt% aqueous solution of sodium hydroxide while cooling. To the solution was added 10.7 g of 2.8 wt% potassium persulfate to prepare a uniform aqueous solution of monomer/initiator.

Separately, 283 ml of cyclohexane was put to a 500 ml flask equipped with a reflux condenser, a dropping funnel, a stirring rod, and a tube for introducing nitrogen, and 3.2 g of lauryldimethylamine oxide was added thereto, followed by stirring at 300 rpm and dispersing. After displacing the inner atmosphere with nitrogen, the mixture was heated to 75° C, and the above-prepared monomer/initiator aqueous solution was added thereto dropwise over a period of 30 minutes. After the addition, the mixture was stirred at 75°C for 1.5 hours and then at 80°C for 4 hours to conduct polymerization.

After completion of the polymerization, the product was collected and dried under reduced pressure to obtain 88.4 g of a sodium acrylate polymer as granular particles. The polymer particles had a distorted shape, a particle size varying from 100 to 1000 µm (average particle size: 720 µm), and a bulk density of 0.6 g/ml. The surface of the individual polymer particle showed extremely remarkable unevenness, looking like irregular-shaped particles of several to 20 µm in diameter fused together.

### EXAMPLE 10

Acrylic acid (72.1 g) was diluted with 18.0 g of water and neutralized with 98.9 g of a 30 wt% aqueous solution of sodium hydroxide while cooling. To the solution was added 10.7 g of 2.8 wt% potassium persulfate to prepare a uniform aqueous solution of monomer/initiator.

Separately, 283 ml of cyclohexane was put to a 500 ml flask equipped with a reflux condenser, a dropping funnel, a stirring rod, and a tube for introducing nitrogen, and 1.1 g of hexadecylethylmethylsulfonium iodide was added thereto, followed by stirring at 300 rpm and dispersing. After displacing the inner atmosphere with nitrogen, the mixture was heated to 75°C, and the above-prepared monomer/initiator aqueous solution was added thereto dropwise over a period of 30 minutes. After the addition, the mixture was stirred at 75°C for 1.5 hours and then at 80°C for 4 hours to conduct polymerization.

After completion of the polymerization, the product was collected and dried under reduced pressure to obtain 88.4 g of a sodium acrylate polymer as granular particles. The polymer particles had a distorted shape, a particle size varying from 200 to 2000 µm (average particle size: 1000 µm), and a bulk density of 0.6 g/ml. The surface of the individual polymer particle showed extremely remarkable unevenness, looking like irregular-shaped particles of 10 to 100 µm in diameter fused together.

### COMPARATIVE EXAMPLE 1

A sodium acrylate polymer was obtained in the same manner as in Example 4, except for using 0.9 g of ethyl cellulose as a dispersant in place of trimethylstearylammonium chloride. The resulting polymer particles had a spherical shape, a particle size of 10 to 400 µm, and a bulk density of 0.94 g/ml. The resulting polymer was tested in the same manner as in Example 4. The results obtained are shown in Table 1.

### COMPARATIVE EXAMPLE 2

A sodium acrylate polymer was obtained in the same manner as in Example 4, except for using 2.9 g of sorbitan monostearate (Leodor SP-S10, produced by Kao Corp. ) as a dispersant in place of trimethylstearylammonium chloride. The resulting polymer was a mixture of spherical particles having a particle size of 10 to 100 µm and secondary particles having a particle size of 50 to 1000 µm, i.e., agglomerates of the spherical particles. The bulk density of the polymer was 0.65 g/ml. When the agglomerates were poured into physiological saline, part of the secondary particles easily disintegrated to primary particles. The resulting polymer was tested in the same manner as in Example 4. The results obtained are shown in Table 1.

### COMPARATIVE EXAMPLE 3

A sodium acrylate polymer was obtained in the same manner as in Example 7, except for using 0.9 g of ethyl cellulose as a dispersant in place of trimethylstearylammonium chloride. The resulting polymer particles had a spherical shape, a particle size of 10 to 400 µm, and a bulk density of 0.93 g/ml. The resulting polymer was tested in the same manner as in Example 4. The results obtained are shown in Table 1.

**TABLE 1**

| | | Equilibrated Water Absorption on Swelling (g/g) | Absorption Representing Rate of Water Absorption (ml/30sec) | Rate of Liquid Permeation (ml/min) |
|---|---|---|---|---|
| Examples | 4 | 58 | 4.1 | 15.5 |
| | 5 | 55 | 4.5 | 11.2 |
| | 6 | 54 | 3.8 | 11.5 |
| | 7 | 55 | 6.8 | 33.4 |
| | 8 | 54 | 6.5 | 28.5 |
| Comparative Examples | 1 | 55 | 2.7 | 2.3 |
| | 2 | 54 | 3.3 | 1.5 |
| | 3 | 52 | 3.6 | 5.4 |

### Industrial Applicability

Because ionic surface active agents generally stabilize an O/W disperse system, they have been used in suspension polymerization or emulsion polymerization of hydrophobic monomers but rarely used in suspension polymerization or emulsion polymerisation of water-soluble monomers. In the present invention, a water-soluble monomer is polymerized in the presence of a cationic or amphoteric surface active agent. While the details of the mechanism are still unclear, considering that ordinary O/W type suspension or emulsion polymerization of a water-soluble monomer produces polymers having a regular spherical shape, it seems that the polymerization in the present invention proceeds in a slightly labile W/O system so that the resulting polymer has many voids and an irregular shape, and still maintains a suitable particle size.

According to the present invention, polymerization of a water-soluble polymerizable monomer in the presence of a cationic or amphoteric surface active agent yields irregular-shaped polymer particles having a small bulk density, excellent in water absorbability, such as initial rate of water absorption, air permeability and liquid permeability, and having excellent gel strength after water absorption and therefore useful as a water-absorbing resin. The process of the present invention is easy to carry out, achieves excellent productivity, and can be applied to mass production.

The irregular-shaped polymer particles obtained by the process of the present invention exhibit particularly excellent water absorbability and are therefore useful as a super absorbent polymer in sanitary products which contact human bodies, such as sanitary napkins, disposable diapers, underwear for adults, tampons, and absorbent wadding. Further, since the polymer hardly undergoes deterioration of its gel structure in long-term use and also has resilience, it is useful as a water-retaining material for horticulture or a water stopping material for civil engineering. The polymer is also expected to be applicable to cosmetic applicators whose shape, resilience, water absorbability, and air permeability are of important.

## Claims

1. A process for producing irregular-shaped polymer particles comprising polymerizing a water-soluble polymerizable monomer by using an aqueous solution of said water-soluble polymerizable monomer and a hydrophobic organic solvent inert to the polymerization, characterized in that the polymerization is carried out in the presence of a cationic or amphoteric surface active agent as a dispersant.

2. The process for producing irregular-shaped polymer particles according to claim 1, characterized in that said dispersant is a surface active agent having an ammonium group.

3. The process for producing irregular-shaped polymer particles according to claim 1, characterized in that said dispersant has a molecular weight of not more than 1000.

4. The process for producing irregular-shaped polymer particles according to claim 2, wherein said surface active agent is a compound represented by formula (I): 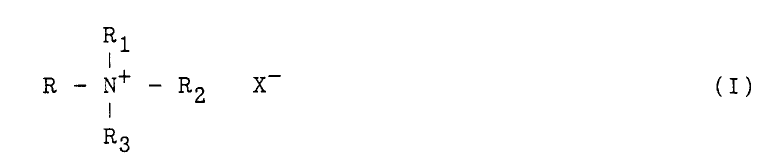 wherein R represents an alkyl, alkenyl or alkylaryl group having 6 or more carbon atoms; R₁, R₂, and R₃ independently represent a hydrogen atom or an alkyl, alkenyl, alkylaryl or hydroxyalkyl group having 1 to 8 carbon atoms, or two or all the groups of R₁, R₂, and R₃ may be connected together to form a saturated or unsaturated ring; and X⁻ represents a halide ion, such as a chloride ion, a bromide ion or an iodide ion, a hydroxide ion (OH⁻) or an acid ion, such as a sulfate ion, an alkylsulfate ion, an alkylsulfonate ion or an organic carboxylate ion.

5. The process for producing irregular-shaped polymer particles according to claim 2, wherein said surface active agent is a compound represented by formula (II): 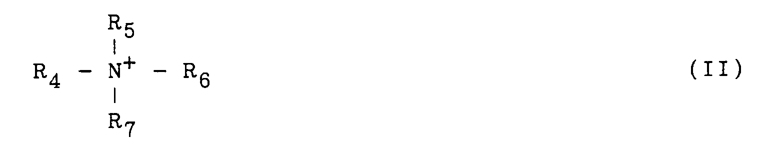 wherein R₄ represents an alkyl, alkenyl or alkylaryl group having 6 or more carbon atoms; R₅ and R₆ independently represent a hydrogen atom or an alkyl, alkenyl, alkylaryl or hydroxyalkyl group having 1 to 8 carbon atoms, or R₅ and R₆ may be connected together to form a saturated or unsaturated ring; and R₇ represents a carboxyalkyl, sulfoalkyl or phosphoalkyl group having 1 to 8 carbon atoms or an oxygen atom.

6. The process for producing irregular-shaped polymer particles according to claim 2, wherein said surface active agent is a compound represented by formula (III): 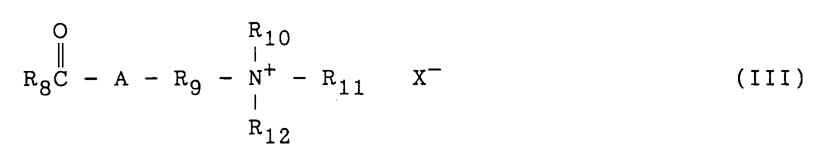 wherein R₈C=O represents an acyl group having 6 or more carbon atoms; A represents -O- or 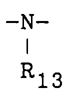 (wherein R₁₃ represents an alkyl group having 1 to 3 carbon atoms); R₉ represents an alkylene group having 1 to 4 carbon atoms; R₁₀, R₁₁, and R₁₂ independently represent a hydrogen atom or an alkyl, alkenyl or alkylaryl group having 1 to 8 carbon atoms, or two or all the groups of R₁₀, R₁₁, and R₁₂ may be connected together to form a saturated or unsaturated ring; and X⁻ represents a halide ion, such as a chloride ion, a bromide ion or an iodide ion, a hydroxide ion (OH⁻) or an acid ion, such as a sulfate ion, an alkylsulfate ion, an alkylsulfonate ion or an organic carboxylate ion.

7. The process for producing irregular-shaped polymer particles according to claim 2, wherein said surface active agent is a compound represented by formula (IV): 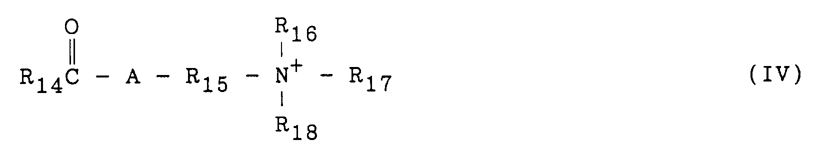 wherein R₁₄C=O represents an acyl group having 6 or more carbon atoms; A represents -O- or 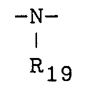 (wherein R₁₉ represents an alkyl group having 1 to 3 carbon atoms); R₁₅ represents an alkylene group having 1 to 4 carbon atoms; R₁₆ and R₁₇ independently represent a hydrogen atom or an alkyl, alkenyl or alkylaryl group having 1 to 8 carbon atoms, or R₁₆ and R₁₇ may be connected together to form a saturated or unsaturated ring; and R₁₈ represents a carboxyalkyl, sulfoalkyl or phosphoalkyl group having 1 to 8 carbon atoms or an oxygen atom.

8. The process for producing irregular-shaped polymer particles according to claim 1, characterized in that said dispersant has a sulfonium group or a phosphonium group and has a molecular weight of not more than 1000.

9. The process for producing irregular-shaped polymer particles according to any of claims 1 to 8, characterized in that said water-soluble polymerizable monomer is an olefinic unsaturated carboxylic acid or an alkali salt thereof.

10. The process according to claim 1, characterized in that said irregular-shaped polymer particle is a water-swellable polymer.

11. The process for producing irregular-shaped polymer particles according to claim 1, characterized in that a crosslinking agent is added to the polymerization system upon polymerizing the water-soluble polymerizable monomer.
